# EUROPEAN PATENT SPECIFICATION

(11) **EP 2 556 059 B1**
(45) Date of publication and mention of the grant of the patent: **07.05.2014**
(21) Application number: 11712864.5
(22) Date of filing: 07.04.2011
(51) Int. Cl.: C07D 257/04, A61K 31/41

(54) **IMPROVED PROCESS FOR PREPARING VALSARTAN**
VERBESSERTES VERFAHREN ZUR HERSTELLUNG VON VALSARTAN
PROCÉDÉ AMÉLIORÉ POUR PRÉPARER DU VALSARTAN

(30) Priority: 07.04.2010 SI 201000116
(43) Date of publication of application: 13.02.2013
(73) Proprietor: KRKA, d.d., Novo mesto, 8501 Novo mesto (SI)
(72) Inventor: ZUPANCIC, Silvo, 8000 Novo mesto (SI)
(74) Representative: UEXKÜLL & STOLBERG
(86) International application number: PCT/EP2011/055451
(87) International publication number: WO 2011/124655

(56) References cited:
- CN-A- 101 450 917
- KR-A- 20090 114 649

## Description

### Field of the Invention

The invention relates to an improved process for the preparation of valsartan wherein the cycloaddition reaction is performed with environmentally less polluting and less dangerous reagents.

### Background of the Invention

Valsartan, N-(1-oxopentyl)-N-[[2'-(1H-tetrazol-5-yl)[1,1'-biphenyl]-4yl]methyl]-L- valine, is a known anti-hypertensive agent having the following formula (I):

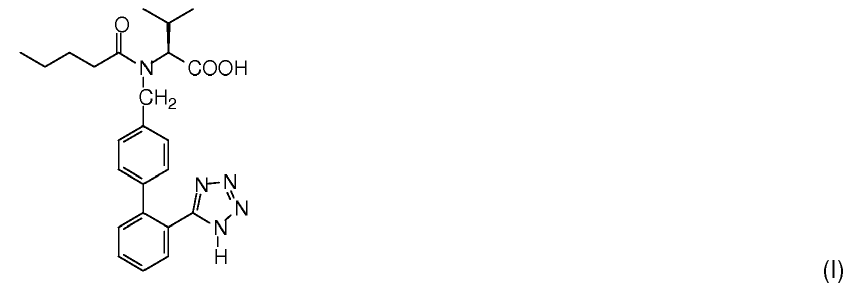

Valsartan is a non-peptide, orally active, specific angiotensin II antagonist, useful in the treatment of hypertension and is commercially available in the market under the brand name DIOVAN^{®} as 40, 80, 160 and 320 mg tablets.

Valsartan, its pharmaceutically acceptable salts, pharmaceutical compositions comprising valsartan and their use in treating high blood pressure and cardiac insufficiency are disclosed in US 5,399,578.

EP 0 443 983, Example 55, discloses a process for the preparation of valsartan benzyl ester wherein 2'-cyanobiphenyl-4-carbaldehyde is condensed with (*L*)-valine benzyl ester hydrochloride in the presence of sodium cyanoborohydride. To the obtained *N*-[(2'-cyanobiphenyl-4-yl)-methyl]-(*L*)-valine benzyl ester valeryl chloride is added, and in the resulting compound the tetrazole moiety is formed using tributyltin azide. In example 54 of this patent, the ester group is removed by catalytic hydrogenation and valsartan is obtained.

In CN 1317485 valsartan is prepared via the acylation of *N*-[(2'-cyano[1,1'-biphenyl]-4-yl)methyl]-L-valine ester hydrochloride salt with pentanoyl chloride in an aromatic solvent in the presence of 4-dimethylaminopyridine and carbonate as catalysts, forming the corresponding pentanoyl ester. Further cyclization with sodium azide in the presence of trialkyltin chloride and polyoxyethylene bis(trimethylsilyl) ether as catalysts yields the product with 55% to 78%.

WO 2007/0114412 discloses a method for the preparation of valsartan comprising the ring closure reaction of the corresponding nitrile in an organic solvent while using alkali or alkaline-earth metal azides or organotin azides, wherein the organic phases containing the nitrile and the tetrazole are first mixed with water while forming three liquid phases after the ring closure reaction has been performed, after which the aqueous phase containing the azide and the phase containing the nitrile are separated, and the middle organic phase containing the tetrazole is processed. As suitable organic solvents toluene, xylene and mesitylene are mentioned.

In WO 2005/014602, a process is described using organo boron and organo aluminium azides for the manufacture of tetrazole derivatives.

WO 2009/001375 describes an improved process wherein substantially pure valsartan (free from tin cations) is prepared employing suitable agents such as chelating agents.

The tetrazole moiety of valsartan can be produced by the reaction of nitrile compounds with azides, such as alkali or alkaline-earth metal or organotin azides, such as trialkyl or triaryltin azides. EP 443983 shows that in the production of valsartan the cycloaddition reaction with sodium or potassium azide and triethyltin, tributyltin or triphenyltin azides is preferred. In the case of valsartan a concluding hydrolysis step is typically required for the production of the desired end product, before the desired product can be obtained as a pure substance or salt. In particular, when using organotin compounds, one should take into consideration that they are highly toxic substances, whose quantitative separation is an essential prerequisite for the applicability of the product obtained and the discarding of which represents an important environmental issue. Dealing with azides in organic solvents further requires a number of safety precautions. In particular, the concluding step of an acidification following hydrolysis can lead to the formation of highly explosive quantities of hydrazoic acid, wherein there is also a great explosion risk in addition to the high toxicity.

In Zhongguo Yiyao Gongye Zazhi, 32(9), 385-387; 2001 the conversion of the CN group to the tetrazole group of valsartan using ZnCl₂/NaN₃ in butanol is described. The drawback of this process is the low yield (40%) and a side reaction, namely esterification of the carboxylic group resulting in the butyl ester of valsartan. This butyl ester is difficult to hydrolyze and therefore a byproduct can be formed such as the R - isomer of valsartan.

In KR 20100055961 similar reaction conditions for the cycloaddition reaction are described. The presence of an alcohol, such as 1-butanol or ethanol, leads to formation of valsartan esters by esterification or transesterification reactions. However, esters of valsartan formed with higher alcohols require stronger reaction conditions for hydrolysis and therefore racemisation of valsartan occurs.

CN 101450917 discloses a process also including use of butanol for forming the tetrazole moiety of valsartan.

KR 10-2010-0132180*,* 10-0995734 *and* 10-1009404 describe the conversion of the CN group to the tetrazole group of valsartan using ZnCl₂/NaN₃ in pyridine. Pyridine is, however, not a user friendly solvent in a big scale production. In addition, its basic character may catalyze racemisation of valsartan or its derivatives.

It is therefore an object of the present invention to provide a process for the preparation of valsartan, and precursors thereof as well as salts of valsartan or of its precursors which process avoids or ameliorates the disadvantages of the prior art and in particular uses less polluting and less dangerous reagents for formation of the tetrazole moiety and results in a small amount of by products and impurities.

### Summary of the invention

According to the present invention, there is provided a process for producing valsartan or a precursor thereof or a salt of valsartan or of a precursor thereof, which comprises reacting, in the presence of a zinc halide and in an ether as reaction solvent, the ether being selected from the group comprising 1,2-diethoxyethane, 1,2-dimethoxyethane, bis(2-methoxyethyl) ether, bis(2-ethoxyethyl) ether or similar solvents having the following structure (II) 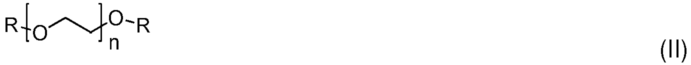 wherein R represents a group chosen among methyl (Me), ethyl (Et), propyl (Pr) or isopropyl (iPr), and n stands for a number chosen among 1, 2, 3, 4, or 5, a 1-cyanobiphenylmethyl derivative of formula (III): 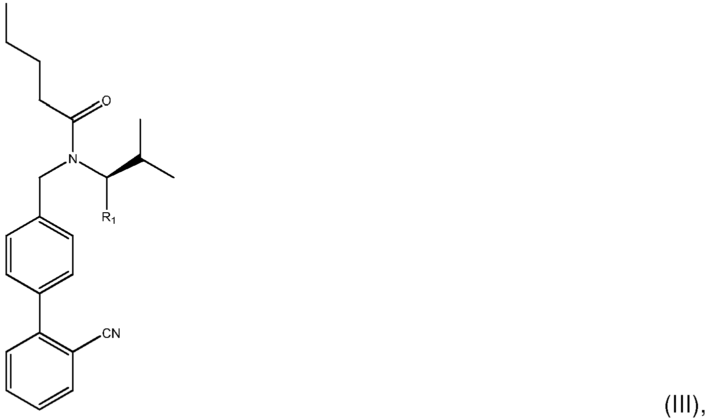 wherein R₁ is CN or COOR₂, R₂ standing for an alkyl, aryl or alkylaryl group such as methyl (Me), ethyl (Et), propyl (Pr), isopropyl (iPr), isobutyl (iBu), sec-butyl (sec-Bu), tert-butyl (t-Bu), or benzyl (Bz), and an inorganic azide salt of formula (IV)

M(N₃)ₘ (IV),

wherein M is an alkali metal or an alkali earth metal, and m is 1 or 2, and optionally hydrolyzing the obtained compound. Optionally, the obtained compound or the compound resulting after the hydrolysis can be converted into a salt thereof.

As zinc halides zinc bromide or zinc chloride may be applied. Preferably, zinc chloride is used.

The hydrolysis is usually effected if the obtained compound is not the desired compound. For example an obtained precursor of valsartan, such as a valsartan ester, can in this way be converted to valsartan.

The hydrolysis of the obtained product to prepare valsartan free acid may be performed using strong alkali metal hydroxides, such as NaOH, KOH or LiOH, after the cycloaddition reaction. The hydrolysis may be effected by addition of an aqueous solution of such a hydroxide. The hydrolysis reaction is preferably carried out at 20°C to 50°C for 1 to 24h. After the hydrolysis reaction is completed, a source of nitrate (III) (i.e. nitrite), preferably NaNO₂, is added to the reaction mixture. The pH value of the mixture is adjusted to a value lower than 2 to catalyze the decomposition of the unreacted azide and to prepare valsartan free acid.

According to a first embodiment, the acid is subsequently extracted with an organic solvent, such as ethyl acetate, isopropyl acetate, dichloromethane or similar solvents. The extract is usually washed with water and concentrated to precipitate crude valsartan. Crude valsartan may be recrystallized from a suitable organic solvent, such as ethyl acetate. In a second embodiment, the prepared valsartan free acid is separated by filtration. The separated wet product is then usually dissolved in an organic solvent, such as ethyl acetate, isopropylacetate, dichloromethane or similar solvents followed by washing the extract with water or diluted acid and concentrating to precipitate crude valsartan.

If desired, the valsartan can also be converted to a salt thereof using conventional techniques.

The 1-cyanobiphenylmethyl derivative of formula (III) may be prepared according to the processes known in the art such as EP 443 983, EP 2090567, or CN101450917.

Also a compound of formula (III) may be prepared from a compound of formula (V) 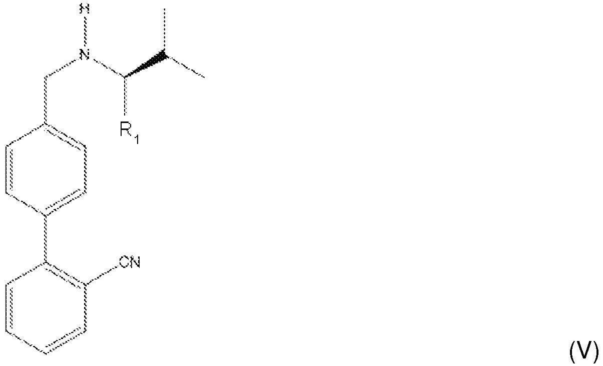 wherein R₁ is CN or COOR₂, R₂ standing for an alkyl, aryl or alkylaryl group such as methyl (Me), ethyl (Et), propyl (Pr), isopropyl (iPr), isobutyl (iBu), sec-butyl (sec-Bu), tert-butyl (t-Bu), or benzyl (Bz), or its salt by reacting with valeroyl chloride in the presence of an aqueous solution of an inorganic base such as NaOH, KOH, Na₂CO₃, K₂CO₃ and an organic solvent not miscible with water such as dichloromethane, toluene, cyclohexane, tert-butyl methyl ether and similar solvents.

### Detailed Description of the Invention

The goal of the invention was to provide a simple, straightforward and environmentally less polluting manufacturing process of valsartan leading to high purity of the final pharmaceutically active product, low cost of the manufacturing process and high chemical yields.

According to the present invention, there is provided a process for producing valsartan and precursors thereof as well as salts of valsartan and of precursors thereof, which comprises reacting, in the presence of a zinc halide and in an ether as reaction solvent, the ether being selected from the group comprising 1,2-diethoxyethane, 1,2-dimethoxyethane, bis(2-methoxyethyl) ether, bis(2-ethoxyethyl) ether or similar solvents having the following structure (II) 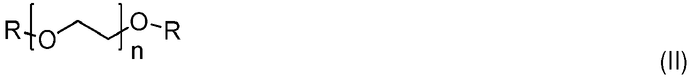 wherein R represents a group chosen among methyl (Me), ethyl (Et), propyl (Pr) or isopropyl (iPr), and n stands for a number chosen among 1, 2, 3, 4 or 5, as the reaction solvent, a 1-cyanobiphenylmethyl derivative of formula (III): 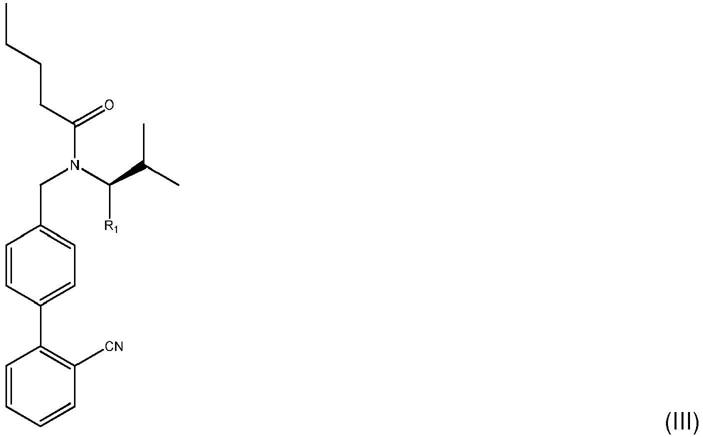 wherein R₁ is CN or COOR₂, R₂ standing for an alkyl, aryl or alkylaryl group such as methyl (Me), ethyl (Et), propyl (Pr), isopropyl (iPr), isobutyl (iBu), sec-butyl (sec-Bu), tert-butyl (t-Bu), or benzyl (Bz), and an inorganic azide salt of formula (IV)

M(N₃)ₘ (IV)

wherein M is an alkali metal or an alkali earth metal, and m is 1 or 2, and optionally hydrolyzing the obtained compound. Optionally, the hydrolyzed or the non-hydrolyzed product can be converted to a salt thereof. As zinc halides zinc bromide or zinc chloride may be applied. Preferably, zinc chloride is used.

Optionally, a phase transfer catalyst may be used in the cycloaddition reaction mixture. Exemplary phase transfer catalysts include, but are not limited to, ammonium salts such as tricaprylylmethylammonium chloride (Aliquat RTM. 336), tetra-n-butylammonium bromide (TBAB), benzyltriethylammonium chloride (TEBA), cetyltrimethylammonium bromide, cetylpyridinium bromide, N-benzylquininium chloride, tetra-n-butylammonium chloride, tetra-n-butylammonium hydroxide, tetra-n-butylarnmonium iodide, tetra-ethylammonium chloride, benzyltributylammonium bromide, benzyltriethylammonium bromide, hexadecyltriethylanmuonium chloride, tetramethylammonium chloride, hexadecyltrimethyl ammonium chloride, octyltrimethylammonium chloride, and combinations comprising one or more of the foregoing catalysts.

Examples of useful inorganic azide salts include azides of alkali metals or alkaline earth metals such as sodium, potassium, lithium, calcium, magnesium, etc. Azides of alkali metals are suitable and sodium azide is industrially more suitable.

The amount of the inorganic azide salt used is usually 1 to 5 moles, preferably 1 to 3 moles, as calculated as hydrogen azide, per mole of nitrile of the formula (III).

Ether solvents preferred in the reaction are selected from the group comprising 1,2-diethoxyethane, 1,2-dimethoxyethane, bis(2-methoxyethyl) ether, bis(2-ethoxyethyl) ether or similar solvents having following structure (II) 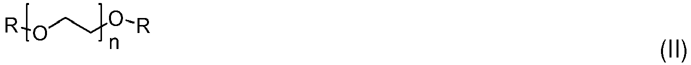 wherein R represents a group chosen among methyl (Me), ethyl (Et), propyl (Pr) or isopropyl (iPr). A mixture of at least two of these solvents can also be used in the reaction. The amount of the solvent used may be a minimum amount which allows the reaction to proceed. The amount of the solvent applied is usually 0.5 to 3 mL, preferably 1 to 1.5 mL, per gram of the nitrile of the formula (III) The ether solvent preferably contains about 5 % or less of water.

The reaction temperature can preferably be selected from a range between 100 °C to 140 °C, preferably from 115 °C to 130 °C, depending on the chosen combination of starting materials, i.e. nitrile and solvent. The reaction time is usually in the range between 15 to 48 hours, preferably from 18 to 30 hours.

The reaction of the present invention proceeds at a high rate, with good to excellent conversion rates, giving only a small amount of byproducts. The reaction ensures high efficiency and can produce the desired product in a higher yield than conventional techniques.

The cyclization reaction may lead to precursors of valsartan differing from valsartan in that they have an R₁ group as defined above for formula (III), in particular a COOR₂ group, instead of the COOH group. The precursor is in particular methyl valsartan or benzyl valsartan, which can easily be converted to valsartan.

When R₁ of formula (III) is a hydrolyzable group and in particular when the group represented by R₂ in the formula (III) has a hydrolyzable group, the hydrolyzable group may be converted to a different group on hydrolysis in the post-treatment after the reaction. For this reaction, in particular bases are used for example aqueous NaOH, KOH and LiOH at a temperature below 45°C. Subsequently, sodium nitrite may be added to the reaction mixture and the pH of the mixture may be adjusted to below 2. During this treatment an excess of azide is decomposed and valsartan is precipitated. The precipitate can either be filtered or dissolved in an organic solvent. In the first case the crude product can for example be treated with ethyl acetate and water at a pH below 2 and then the organic phase can be concentrated and the precipitated product can be separated. Employing such isolation procedure, results in the level of residual reagents and starting materials (Zn compounds, azide, and valeric acid) used in the process to be very low. In particular, Zn compounds are below 5 ppm, azide compounds are below 10 ppm and valeric acid is below 325ppm. In the second case the organic phase is washed with water and concentrated and the precipitated product is separated, for example by filtration.

The organic solvent may be chosen from the group of solvents including: ethyl acetate, isopropyl acetate, butyl acetate, isobutyl acetate, *tert-*butyl acetate, dichloromethane, toluene, cyclohexane, and tert-butyl methyl ether. Preferably ethyl acetate and isopropyl acetate are used.

As pointed out above, the hydrolysis of the obtained product to prepare valsartan may be performed using strong alkali metal hydroxides such as NaOH, KOH, LIOH. This may be carried out by addition of an aqueous solution of such hydroxide to the reaction mixture after the cycloaddition reaction. The hydrolysis reaction is preferably performed at 20 °C to 50°C for 1 to 24 hours. After the reaction is completed, NaNO₂ is usually added to the reaction mixture. The pH of the mixture is adjusted to below 2 to catalyze decomposition of unreacted azide and to prepare valsartan free acid. This acid is usually extracted with organic solvent such as ethyl acetate, isopropyl acetate, dichloromethane and or similar solvents. The extract is then usually washed with water and concentrated to precipitate crude valsartan. The crude valsartan may then be recrystallized from an organic solvent such as ethyl acetate.

The 1-cyanobiphenylmethyl derivative of formula (III) may be prepared according to any of the processes known in the art or obvious to the person skilled in the art. The compounds of formula (III) may be prepared from a compound of formula (V) 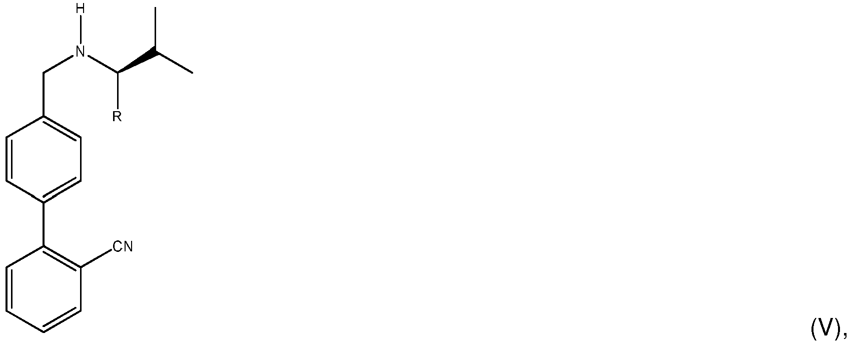 wherein R₁ is CN or COOR₂, R₂ standing for an alkyl, aryl or alkylaryl group such as methyl (Me), ethyl (Et), propyl (Pr), isopropyl (iPr), isobutyl (iBu), sec-butyl (sec-Bu), tert-butyl (t-Bu), or benzyl (Bz), or its salt by reacting with valeroyl chloride in the presence of an aqueous solution of an inorganic base such as NaOH, KOH, Na₂CO₃, K₂CO₃ and an organic solvent not miscible with water such as dichloromethane, toluene, cyclohexane, tert-butyl methyl ether and similar solvents.

In particular the present invention provides the following items:
1.) A process for producing valsartan or a precursor thereof or a salt of valsartan or of a precursor thereof, which comprises reacting, in the presence of a zinc halide and in an ether as reaction solvent, the ether being selected from the group comprising 1,2-diethoxyethane, 1,2-dimethoxyethane, bis(2-methoxyethyl) ether, bis(2-ethoxyethyl) ether or similar solvents having the following structure (II) 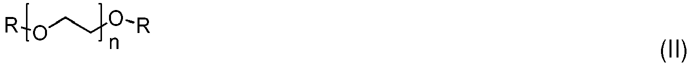 wherein R represents a group chosen among methyl (Me), ethyl (Et), propyl (Pr) or isopropyl (iPr), and n stands for a number chosen among 1,2,3,4, or 5, a 1-cyanobiphenylmethyl derivative of formula (III): 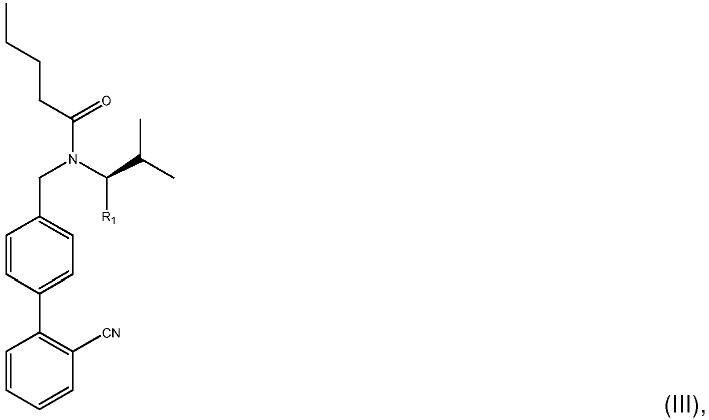 wherein R₁ is CN or COOR₂, R₂ standing for an alkyl, aryl or alkylaryl group such as methyl (Me), ethyl (Et), propyl (Pr), isopropyl (iPr), isobutyl (iBu), sec-butyl (sec-Bu), tert-butyl (t-Bu), or benzyl (Bz), and an inorganic azide salt of formula (IV)

   M(N₃)ₘ (IV)

   wherein M is an alkali metal or an alkali earth metal, and m is 1 or 2, and optionally hydrolyzing the obtained compound.
2.) A process according to item 1 characterized in that the zinc halide is chosen among zinc chloride and zinc bromide.
3.) A process according to any of items 1 and 2, characterized in that zinc chloride is used.
4.) A process according to any of items 1 to 3, characterized in that the amount of the solvent applied is between 0.5 to 3 mL, preferably between 1 to 1.5 mL, per gram of the nitrile of the formula (II).
5.) A process according to any of items 1 to 4, characterized in that the reaction temperature is selected from a range between 100 °C to 140 °C, preferably from a range between 115 °C to 130 °C.
6.) A process according to any of items 1 to 5, characterized in that the hydrolysis is performed using strong alkali metal hydroxides at 20 °C to 50 °C and in that the excess azide is decomposed at a pH value lower than 2 and in the presence of a nitrate (III) source.
7.) A process according to item 6, characterized in that the nitrate (III) source is sodium nitrate (III).

The invention is further illustrated by the following examples which demonstrate the preparation of valsartan without being limited thereto. Parts or percentages are based on weight, unless specified otherwise. The products were analyzed according to Ph. Eur.

### Examples

### Example 1

To 6.91 g (15 mmol) of (S)-methyl 2-(N-((2'-cyano-[1,1'-biphenyl]-4-yl)methyl)pentanamido)-3-methylbutanoate in form of colorless oil, 4.71 g (72 mmol) of sodium azide, 3.66 g (27 mmol) of ZnCl₂ and 7 mL diglyme were added. The mixture was heated for 26 hours at 120 °C.
Area% HPLC purity of the reaction mixture: content of the product (methyl valsartan) 89.3 %, content of the nitrile starting material: 1.6 %.

The reaction mixture was cooled and 65 mL of ethyl acetate were added. The organic phase was washed with water (20 mL, 3x) and evaporated. To the residue 40 mL of 3M NaOH were added and the mixture was heated at 40 °C for 4 hours. The mixture was then cooled and the pH value of the mixture was adjusted to a value between 1.5 to 2.0. 50 mL of ethyl acetate were added and the phases were stirred for 5 min. Then the phases were separated and the aqueous phase was re-extracted with 50 mL of ethyl acetate. The collected organic phases were washed with ethyl acetate (20 mL 3x) and evaporated. To the concentrate 100 mL of ethyl acetate were added and the mixture was evaporated again. To the residue 40 mL of ethyl acetate were added and the mixture was stirred at room temperature for 24 h. During this time crude valsartan precipitated. The obtained suspension was cooled to a temperature below 0 °C and stirred at this temperature for 1 h and then the product was separated by filtration and dried.
Yield: 5.7g (87 %) of crude valsartan.
The crude product was recrystallized from ethyl acetate.
Overall yield: 4.9 g (86 %).
Content of individual impurities: below 0.10 %.

### Example 2

To 6.91 g (15 mmol) of (S)-methyl 2-(N-((2'-cyano-[1,1'-biphenyl]-4-yl)methyl)pentanamido)-3-methylbutanoate in form of colorless oil, 4.2 g (65 mmol) of sodium azide, 3.2 g (24 mmol) of ZnCl₂ and 6 mL diglyme were added. The mixture was heated for 26 hours at 120 °C.
Area% HPLC purity of the reaction mixture: content of the product (methyl valsartan) 92.6 %, content of the nitrile starting material: 0.6 %.

The reaction mixture was cooled and 65 mL of ethyl acetate were added. The organic phase was washed with water (20 mL, 3x) and evaporated. To the residue 40 mL of 3M NaOH were added and the mixture was heated at 40 °C for 4 hours. Then the mixture was cooled and the pH value of the mixture was adjusted to a value between 1.5 to 2.0. After that 50 mL of ethyl acetate were added and the phases were stirred for 5 min. Then the phases were separated and the aqueous phase was re-extracted with 50 mL of ethyl acetate. The collected organic phases were washed with ethyl acetate (20 mL 3x) and evaporated. To the concentrate 100 mL of ethyl acetate were added and the mixture was evaporated again. To the residue 40 mL of ethyl acetate were added and the mixture was stirred at room temperature for 24 h. During this time crude valsartan precipitated. The obtained suspension was cooled to a temperature below 0 °C and stirred at this temperature for 1 h and then the product was separated by filtration and dried.
Yield: 5.8g (89 %) of crude valsartan.
The crude product was re-crystallized from ethyl acetate.
Overall yield: 5.1 g (88 %).
Content of individual impurities: below 0.10 %.

### Example 3

To 6.91 g (15 mmol) of (S)-methyl 2-(N-((2'-cyano-[1,1'-biphenyl]-4-yl)methyl)pentanamido)-3-methylbutanoate in form of a colorless oil, 4.71 g (72 mmol) of sodium azide, 3.66 g (27 mmol of ZnCl₂) and 6 mL ethyl diglyme were added. The mixture was heated for 26 hours at 120 °C.
Area% HPLC purity of the reaction mixture: content of the product (methyl valsartan) 87.5 %, content of the nitrile starting material: 2.5 %.

### Example 4

a.) To the mixture of 170 ml of dichloromethane, 115 ml 25% K₂CO₃ and 17 g (47 mmol) methyl 2-(((2'-cyano-[1,1'-biphenyl]-4-yl)methyl)amino)-3-methylbutanoate hydrochloride at 0°C to 5°C 8.2 ml (68 mmol) of valeroyl chloride was slowly added and the mixture was stirred at this temperature for 5.5 hours. Then the phases were separated and the organic phase was washed with 16 ml 1 M HCl and 16 ml of saturated solution of NaHCO₃ and 16 ml of water. The organic phase was then evaporated to obtain (S)-methyl 2-(N-((2'-cyano-[1,1'-biphenyl]-4-yl)methyl)pentanamido)-3-methylbutanoate as an oil.
b.) To 6.91 g (15 mmol) of (S)-methyl 2-(N-((2'-cyano-[1,1'-biphenyl]-4-yl)methyl)pentanamido)-3-methylbutanoate in form of a colorless oil, 4.71 g (72 mmol) of sodium azide, 3.66 g (27 mmol) of ZnCl₂ and 7 mL diglyme were added. The mixture was heated for 26 hours at 120 °C.
   Area% HPLC purity of the reaction mixture: content of the product (methyl valsartan) 89.3 %, content of the nitrile starting material: 1.6 %.

The reaction mixture was cooled to below 40°C and then 14 mL of 2M NaOH were added and it was stirred at room temperature for 3 hours. Then 2.39 g of sodium nitrite were added and the pH of the mixture was adjusted to 1.6 by addition of 6M HCl. After that 50 mL of ethyl acetate were added and the mixture was stirred for 5 min. Phases were separated and the aqueous phase was re-extracted with 50 mL of ethyl acetate. The collected organic phases were washed with ethyl acetate (20 mL 3x) and evaporated. To the concentrate 100 mL of ethyl acetate were added and the mixture was evaporated again. Then 40 mL of ethyl acetate were added and the mixture was stirred at room temperature for 24 h. During this time crude valsartan precipitated. The obtained suspension was cooled to a temperature below 0°C and stirred at this temperature for 1 h and then the product was separated by filtration and dried.
Yield: 5.85 g (90 %) of crude valsartan.
The crude product was re-crystallized from ethyl acetate.
Overall yield of the synthesis: 81 %
Content of individual impurities: Below 0.10 %

### Example 5

To 18.3 g (44 mmol) of (S)-methyl 2-(N-((2'-cyano-[1,1'-biphenyl]-4-yl)methyl)pentanamido)-3-methylbutanoate in form of a colorless oil, 17 mL diglyme 11.3 g (174 mmol) of sodium azide and 8.57 g (63 mmol) of ZnCl₂ were added. The mixture was heated for 24 hours at 120 °C.
Area% HPLC purity of the reaction mixture: content of the product (together methyl valsartan and valsartan) 90.1 %, content of the nitrile starting material: 2.1 %.

The reaction mixture was cooled to below 40°C and then 20 ml of water and 125mL of 2M NaOH were added and stirred at room temperature overnight. Then 11.2 g of sodium nitrite were added and the pH of the mixture was adjusted to below 1 by addition of concentrated HCl. The precipitate was filtered and then 270 ml of ethyl acetate and 150 ml of water were added. The pH value was adjusted to below 1 by addition of aqueous HCl and the mixture was stirred for 5 min. Phases were separated and the organic phase was washed with water (190 mL 4x). The pH value of the aqueous phases was adjusted to below 1 during each washing. The organic phase was evaporated at temperature below 40 °C to give a thick residue (200g). The residue was stirred at room temperature for 24 h. During this time crude valsartan precipitated. The obtained suspension was cooled to a temperature below 0°C and stirred at this temperature for 1 h and then the product was separated by filtration and dried.
Yield: 15.3 g (80 %) of crude valsartan.
The crude product was re-crystallized from ethyl acetate.
Overall yield of the synthesis: 74%
Content of individual impurities after recrystallization: Below 0.10 %

### Comparative Example 1

To 45 mmol of (S)-methyl 2-(N-((2'-cyano-[1,1'-biphenyl]-4-yl)methyl)pentanamido)-3-methylbutanoate dissolved in xylene 6.28 g (96.8 mmol) of sodium azide and 20.5 ml (75.5 mmol) tributyltin chloride were added. The mixture was heated for 44 hours at 138 °C.
Area% HPLC purity of the reaction mixture: content of the product (methyl valsartan) 88.5 %, content of the nitrile starting material: 2.7 %.

### Comparative Example 2

To 5 mmol of (S)-methyl 2-(N-((2'-cyano-[1,1'-biphenyl]-4-yl)methyl)pentanamido)-3-methylbutanoate dissolved in 10 ml of ethyleneglycol (ethane-1,2-diol) 1.3 g (20 mmol) of NaN₃ and 1.49 g (11 mmol) ZnCl₂ were added. The mixture was heated for 23 hours at 120 °C.

Area% HPLC purity of the reaction mixture: content of the product (methyl valsartan and valsartan) 20 %, content of the nitrile starting material: 0.2 %.

### Comparative Example 3

To 5 mmol of (S)-methyl 2-(N-((2'-cyano-[1,1'-biphenyl]-4-yl)methyl)pentanamido)-3-methylbutanoate dissolved in 10 ml of isoamylalcohol (3-methyl-1-butanol) 1.3 g (20 mmol) of NaN₃ and 1.49 g (11 mmol) ZnCl₂ were added. The mixture was heated for 23 hours at 120 °C.

Area% HPLC purity of the reaction mixture: content of the product (methyl valsartan and valsartan) 67 %, content of the nitrile starting material: 0.5 %.

### Comparative Example 4

10g of (S)-methyl 2-(*N*-((2'-cyano-[1,1'-biphenyl]-4-yl)methyl)pentanamido)-3-methylbutanoate were dissolved in 50 ml of 1-butanol and then 11.7g of zinc chloride and 8g of sodium azide were added. The mixture was stirred at reflux temperature for 20h.

Area% HPLC of the reaction mixture: content of the product (methyl valsartan and valsartan) 69.9 %, content of the nitrile starting material: 1.1 %.

## Claims

1. A process for producing valsartan, or a precursor thereof having the formula 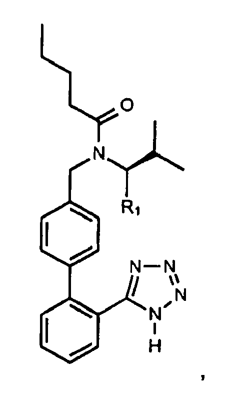 wherein R₁ is CN or COOR₂, R₂ standing for an alkyl, aryl or alkylaryl group such as methyl (Me), ethyl (Et), propyl (Pr), isopropyl (iPr), isobutyl (iBu), sec-butyl (sec-Bu), tert-butyl (t-Bu), or benzyl (Bz),
or a salt of valsartan or of the precursor thereof, which comprises reacting, in the presence of a zinc halide and in an ether as reaction solvent, the ether being selected from the group comprising 1,2-diethoxyethane, 1,2-dimethoxyethane, bis(2-methoxyethyl) ether, bis(2-ethoxyethyl) ether or similar solvents having the following structure (II) 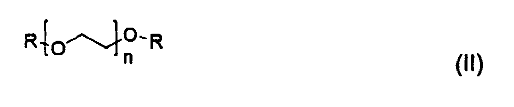 wherein R represents a group chosen among methyl (Me), ethyl (Et), propyl (Pr) or isopropyl (iPr), and n stands for a number chosen among 1,2,3,4, or 5,
a 1-cyanobiphenylmethyl derivative of formula (III): 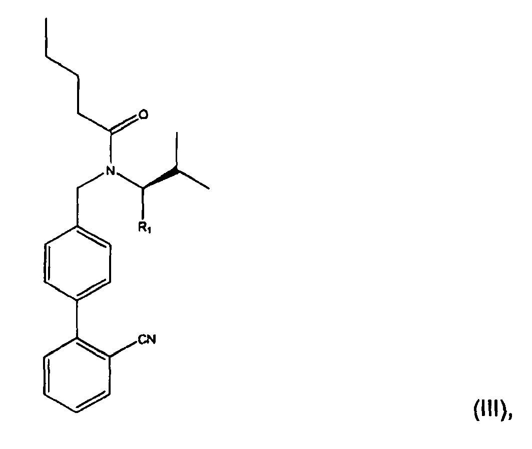 and an inorganic azide salt of formula (IV)
M(N₃)ₘ (IV)
wherein M is an alkali metal or an alkali earth metal, and m is 1 or 2, and optionally hydrolyzing the obtained compound.

2. A process according to claim 1 **characterized in that** the zinc halide is chosen among zinc chloride and zinc bromide.

3. A process according to any of claims 1 and 2, **characterized in that** zinc chloride is used.

4. A process according to any of claims 1 to 3, **characterized in that** the amount of the solvent applied is between 0.5 to 3 mL per gram of the nitrile of the formula (III).

5. A process according to claim 4, **characterized in that** the amount of the solvent applied is between 1 to 1.5 mL per gram of the nitrile of the formula (III).

6. A process according to any of claims 1 to 5, **characterized in that** the reaction temperature is selected from a range between 100 °C to 140 °C.

7. A process according to claim 6, **characterized in that** the reaction temperature is selected from a range between 115 °C to 130 °C.

8. A process according to any of claims 1 to 7, **characterized in that** the hydrolysis is performed using strong alkali metal hydroxides at 20 °C to 50 °C and **in that** the excess azide is decomposed at a pH value lower than 2 and in the presence of a nitrite source.

9. Process according to claim 8, wherein after hydrolysis and decomposing of excess azide, valsartan is precipitated and the precipitate is treated with an organic solvent and water at a pH of below 2, and valsartan is recovered from the organic phase.

10. Process according to claim 9, wherein the organic solvent is ethylacetate.

11. A process according to any one of claims 8 to 10, **characterized in that** the nitrite source is sodium nitrite.

12. A process according to any one of claims 1 to 11, wherein the precursor of valsartan is methyl valsartan or benzyl valsartan.

## Patentansprüche

1. Verfahren zur Herstellung von Valsartan oder einem
Vorläufer davon mit der Formel 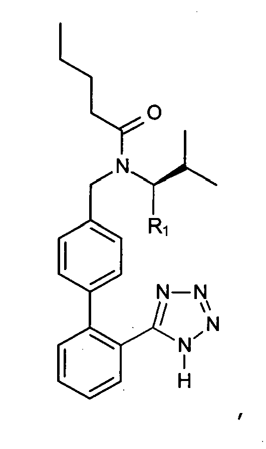 in der R₁ CN oder COOR₂ ist und R₂ für eine Alkyl-, Aryl- oder Alkylaryl-Gruppe steht, wie z.B. Methyl (Me), Ethyl (Et), Propyl (Pr), Isopropyl (iPr), Isobutyl (iBu), sec-Butyl (sec-Bu), tert-Butyl (t-Bu) oder Benzyl (Bz),
oder einem Salz von Valsartan oder dem Vorläufer davon, bei dem in Gegenwart eines Zinkhalogenids und in einem Ether als Reaktionslösungsmittel, wobei der Ether ausgewählt ist aus der Gruppe aus 1,2-Diethoxyethan, 1,2-Dimethoxyethan, bis(2-Methoxyethyl)ether, bis(2-Ethoxyethyl)ether oder ähnlichen Lösungsmitteln mit der folgenden Struktur (II) 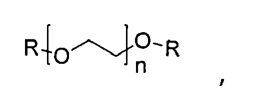 in der R eine Gruppe ausgewählt aus Methyl (Me), Ethyl (Et), Propyl (Pr) oder Isopropyl (iPr) darstellt und n für eine Zahl ausgewählt aus 1, 2, 3, 4 oder 5 steht,
ein 1-Cyanobiphenylmethylderivat der Formel (III): 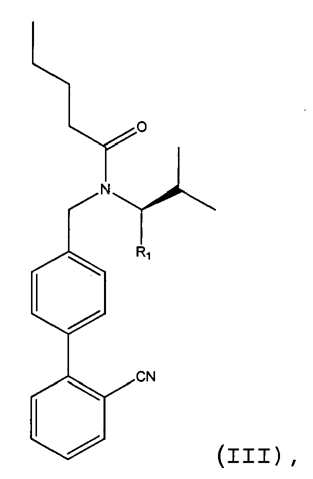 und ein anorganisches Azidsalz der Formel (IV)
M(N₃)ₘ (IV),
in der M ein Alkalimetall oder ein Erdalkalimetall ist und m 1 oder 2 ist, zur Reaktion gebracht werden und gegebenenfalls die erhaltene Verbindung hydrolysiert wird.

2. Verfahren nach Anspruch 1, **dadurch gekennzeichnet, dass** das Zinkhalogenid aus Zinkchlorid und Zinkbromid ausgewählt ist.

3. Verfahren nach einem der Ansprüche 1 und 2, **dadurch gekennzeichnet, dass** Zinkchlorid verwendet wird.

4. Verfahren nach einem der Ansprüche 1 bis 3, **dadurch gekennzeichnet, dass** die Menge des angewendeten Lösungsmittels zwischen 0,5 bis 3 ml/g des Nitrils der Formel (III) beträgt.

5. Verfahren nach Anspruch 4, **dadurch gekennzeichnet, dass** die Menge des verwendeten Lösungsmittels zwischen 1 bis 1,5 ml/g des Nitrils der Formel (III) beträgt.

6. Verfahren nach einem der Ansprüche 1 bis 5, **dadurch gekennzeichnet, dass** die Reaktionstemperatur aus einem Bereich zwischen 100 bis 140°C ausgewählt ist.

7. Verfahren nach Anspruch 6, **dadurch gekennzeichnet, dass** die Reaktionstemperatur aus einem Bereich zwischen 115 bis 130°C ausgewählt ist.

8. Verfahren nach einem der Ansprüche 1 bis 7, **dadurch gekennzeichnet, dass** die Hydrolyse unter Verwendung starker Alkalimetallhydroxide bei 20 bis 50°C durchgeführt wird und dass der Überschuss Azid bei einem pH-Wert von weniger als 2 und in der Gegenwart einer Nitritquelle zersetzt wird.

9. Verfahren nach Anspruch 8, bei dem nach Hydrolyse und Zersetzung des Überschusses von Azid Valsartan ausgefällt wird und die Ausfällung mit einem organischen Lösungsmittel und Wasser bei einem pH-Wert von weniger als 2 behandelt und Valsartan aus der organischen Phase gewonnen wird.

10. Verfahren nach Anspruch 9, bei dem das organische Lösungsmittel Ethylacetat ist.

11. Verfahren nach einem der Ansprüche 8 bis 10, **dadurch gekennzeichnet, dass** die Nitritquelle Natriumnitrit ist.

12. Verfahren nach einem der Ansprüche 1 bis 11, bei dem der Vorläufer von Valsartan Methylvalsartan oder Benzylvalsartan ist.

## Revendications

1. Procédé de préparation de valsartan, ou d'un précurseur de
valsartan de formule 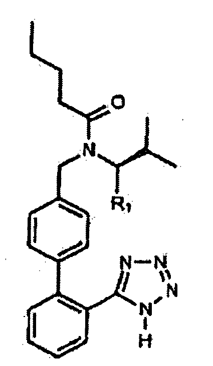 dans laquelle R₁ représente un groupe de formule CN ou COOR₂ où R₂ représente un groupe alkyle, aryle ou aryl-alkyle, comme un groupe méthyle (Me), éthyle (Et), propyle (Pr), isopropyle (iPr), isobutyle (iBu), sec-butyle (sBu), tertiobutyle (tBu) ou benzyle (Bz),
ou d'un sel de valsartan ou de précurseur de valsartan,
lequel procédé comporte le fait de faire réagir, en présence d'un halogénure de zinc et dans un éther servant de solvant de réaction, lequel éther est choisi dans l'ensemble comprenant les 1,2-diéthoxy-éthane, 1,2-diméthoxy-éthane, bis(2-méthoxy-éthyl)éther, bis(2-éthoxy-éthyl)-éther et solvants similaires de structure (II) suivante : 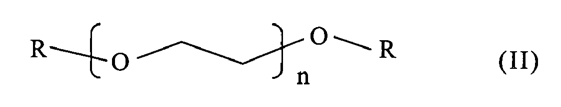 dans laquelle R représente un groupe choisi parmi les groupes méthyle (Me), éthyle (Et), propyle (Pr) et isopropyle (iPr), et l'indice n est un nombre choisi parmi 1, 2, 3, 4 et 5,
un dérivé à groupe (1-cyano-biphénylyl)-méthyle, de formule (III) : 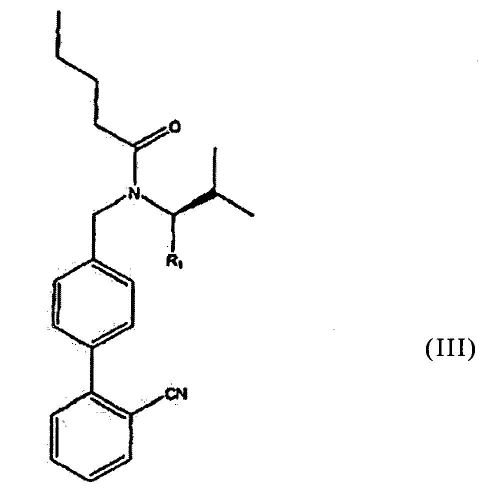 et un sel de type azoture inorganique, de formule (IV) :
M(N₃)ₘ (IV)
dans laquelle M représente un atome de métal alcalin ou alcalino-terreux et l'indice m vaut 1 ou 2,
et en option, le fait d'hydrolyser le composé obtenu.

2. Procédé conforme à la revendication 1, **caractérisé en ce que** l'halogénure de zinc est choisi parmi le chlorure de zinc et le bromure de zinc.

3. Procédé conforme à l'une des revendications 1 et 2, **caractérisé en ce que** c'est du chlorure de zinc qui est utilisé.

4. Procédé conforme à l'une des revendications 1 à 3, **caractérisé en ce que** l'on utilise le solvant en une proportion de 0,5 à 3 mL par gramme du nitrile de formule (III).

5. Procédé conforme à la revendication 4, **caractérisé en ce que** l'on utilise le solvant en une proportion de 1 à 1,5 mL par gramme du nitrile de formule (III).

6. Procédé conforme à l'une des revendications 1 à 5, **caractérisé en ce que** la réaction se déroule à une température choisie dans la gamme allant de 100 à 140 °C.

7. Procédé conforme à la revendication 6, **caractérisé en ce que** la réaction se déroule à une température choisie dans la gamme allant de 115 à 130 °C.

8. Procédé conforme à l'une des revendications 1 à 7, **caractérisé en ce que** l'on effectue l'hydrolyse en utilisant une base forte de type hydroxyde de métal alcalin, à une température de 20 à 50 °C, et **en ce que** l'on décompose l'excès d'azoture à un pH inférieur à 2 et en présence d'une source de nitrite.

9. Procédé conforme à la revendication 8, dans lequel, après l'hydrolyse et la décomposition de l'excès d'azoture, on fait précipiter le valsartan et l'on traite le précipité avec un solvant organique et de l'eau, à un pH inférieur à 2, et l'on récupère le valsartan dans la phase organique.

10. Procédé conforme à la revendication 9, dans lequel le solvant organique est de l'acétate d'éthyle.

11. Procédé conforme à l'une des revendications 8 à 10, **caractérisé en ce que** la source de nitrite est du nitrite de sodium.

12. Procédé conforme à l'une des revendications 1 à 11, dans lequel le précurseur de valsartan est le méthyl-valsartan ou le benzylvalsartan.
